Europäisches Patentamt

European Patent Office

Office européen des brevets

(11) Publication number: **0 209 804**
**A2**

# EUROPEAN PATENT APPLICATION

(21) Application number: 86109450.6

(22) Date of filing: 10.07.86

(51) Int. Cl.⁴: **A 61 B 5/07**
**A 61 B 5/04, A 61 B 5/02**

(30) Priority: 25.07.85 IT 6768785

(43) Date of publication of application:
28.01.87 Bulletin 87/5

(84) Designated Contracting States:
AT BE CH DE FR GB LI NL SE

(71) Applicant: Rossi, Paolo
Via Verdi, 18
28100 Novara(IT)

(72) Inventor: Rossi, Paolo
Via Verdi, 18
28100 Novara(IT)

(74) Representative: Prato, Roberto et al,
c/o Ingg. Carlo e Mario Torta Via Viotti 9
I-10121 Torino(IT)

(54) Unit with a subcutaneous pick-up for continuously monitoring physiological cardiorespiratory variables.

(57) Unit for continuously monitoring and detecting variations in physiological cardiorespiratory variables, such as electrocardiogram signals (QRS-T), heart rate, ventilation/minute (VE), breathing rate, systolic output (SO), cardiac output (CO) and arterial pressure (AP).

The said unit comprises a first subcutaneous part (1) having means (6, 9, 12) for detecting first physiological parameters, such as the difference in potential between electrodes, transthoracic impedance and subcutaneous blood pressure, and means for handling first signals, depending on the said physiological parameters, and for transmitting them, by radio-frequency, to a second part (18) outside the body; the latter part having means for receiving the said physiological variables, which are then processed for detecting critical conditions and activating alarm means (35, 36).

EP 0 209 804 A2

./...

Fig.1a

Fig.1b

UNIT WITH A SUBCUTANEOUS PICK-UP FOR CONTINUOUSLY
MONITORING PHYSIOLOGICAL CARDIORESPIRATORY VARIABLES

The present invention relates to a unit with a subcutaneous pick-up for continuously monitoring physiological
cardiorespiratory variables.

For continuously monitoring the cardiorespiratory condition of patients, essentially by means of an electrocardiogram signal, such control is currently known to be
conducted using external equipment, the pick-up electrodes of which are arranged on the outside of the patient's body. This limits, not only the scope of the
control itself, which may only be performed for limited
periods of time, but also the activity of the patient,
on account of the pick-up equipment being applied to
the outside of the patient's body.

The aim of the present invention is to provide a unit
for continuously monitoring cardiorespiratory functions,
which involves no limitation in the activity of the patient, and which provides for automatically detecting critical conditions and activating an alarm.

Further aims and advantages of the unit according to the present invention will be disclosed in the following description.

With this aim in view, the present invention relates to a unit for continuously monitoring physiological cardiorespiratory variables, characterised by the fact that it comprises a first subcutaneous part having means for detecting first physiological parameters, and means for handling the said first parameters and transmitting relative first signals to a second part outside the patient's body; the latter part having means for handling the said first signals, and means for processing the said second physiological variables and/or variations in the same, received from the said handling means, for detecting critical conditions and activating alarm means.

A non-limiting arrangement of the present invention will now be described with reference to the attached drawings in which :

- Fig.s 1a, 1b and 1c show views in perspective of three parts on the unit according to the present invention;

- Fig.s 2a and 2b show respective block diagrams of the parts shown in Fig.s 1a and 1b.

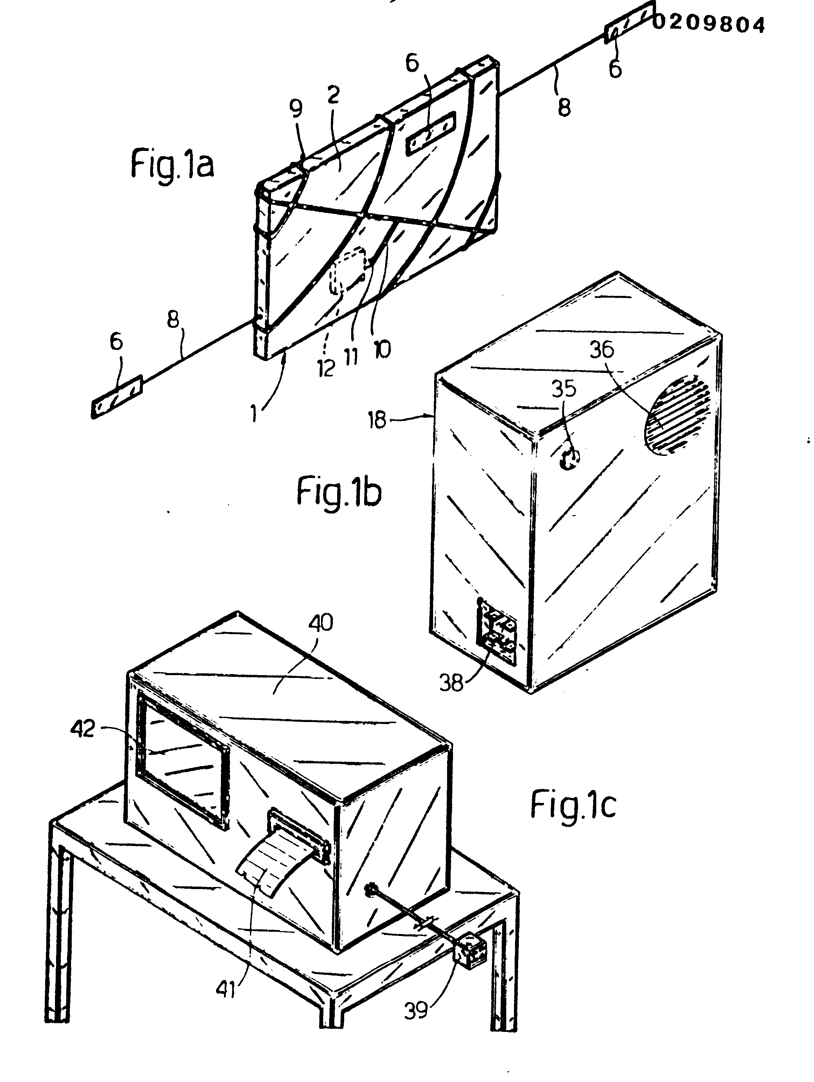

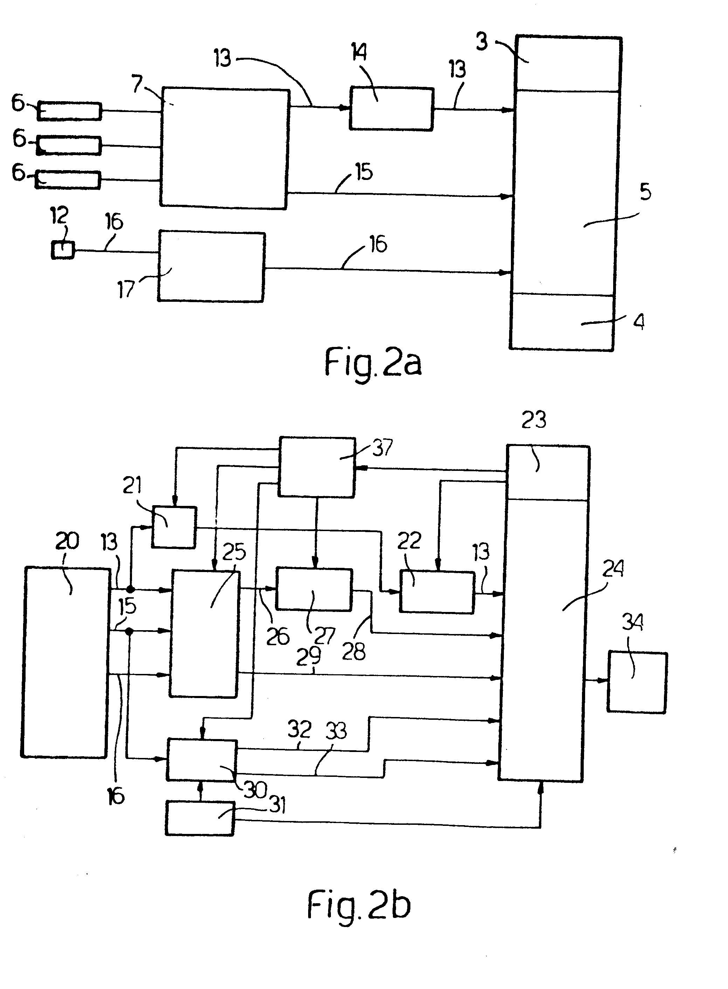

Fig.1a shows a first part 1 of the unit according to the present invention, the said part consisting of a rectangular case 2 conveniently titanium-plated, a few centimetres in length and width, a few millimetres thick and containing : circuitry described in more detail later on with reference to Fig. 2a, a solid-state, conveniently lithium battery 3 having a supply life of a few years for supplying the circuitry on part 1, a quartz clock 4 and a radio-frequency transmitter 5. The said first part 1 presents three electrodes 6 connected to an internal circuit block 7. Of the said three electrodes 6, one is affixed to case 2 and may conveniently be formed from a portion of the same, whereas the other two are connected by 7-10 cm long wires 8 through case 2, with organic liquid sealing portions conveniently formed of silicone. About case 2 is wound a wide-mesh network 9 of glycerine-filled, intercommunicating teflon tubes, one branch 10 of which fits inside case 2, with an organic liquid insulating zone 11, so as to communicate hydraulically with an electric pressure microtransducer 12 inside part 1. The latter is inserted under the skin, conveniently in the left armpit or mammary region, between the third and fifth rib space, the said electrode 6 affixed to case 2 being arranged facing the ribs, and the other two electrodes 6 being positioned so as to pick up an electrocardiogram signal from the front and lower-rear surfaces of the heart. Block 7 (Fig. 2a) detects the difference in potential between each of the two detached electrodes 6 and the one affixed to case 2, so as to pick up an electrocardiogram signal from two simultaneous branches. These are then processed and sent, in the form of electrocardiogram signal 13, to an amplifying block 14 the output signal of which is then sent to transmitting block 5. Block 7 also detects variations in transthoracic impedance between two of the said three electrodes 6, and supplies a signal 15 also to transmitter 5. As described in more detail later on, variations in transthoracic impe-

dance signal 15 are used for measuring variations in respiratory ventilation/minute (VE) (breathing rate times current volume) and in systolic output (SO). These two parameters, in fact, differ as to frequency and length : impedance variations caused by breathing are slow and independant of cardiac activity, whereas those caused by systolic output are synchronous with the QRS section on the electrocardiogram signal. Microtransducer 12, which may be a piezoelectric crystal or electromagnetic induction type, supplies a signal 16 depending on the pressure and pressure variations of network 9. Via an amplifying block 17, the said signal 16 is supplied to transmitting block 5 which also comprises a transmission band and time constant selecting circuit. The said transmission band may conveniently range from 5 to 1000 Hertz, in that the frequency of electrocardiogram signal section QRS ranges from 40 to 100 Hertz, that of section S-T from 5 to 40 Hertz, whereas transthoracic impedance may vary by as much as 10 Hertz.

The unit according to the present invention also comprises a second part 18 (Fig. 1b) in the form of a portable pocket box and having internal circuitry as shown on the block diagram in Fig. 2b. The latter shows a receiving block 20 for receiving, even over a distance of a few metres, the signals transmitted by transmitting block 5 on part 1, which separates, by means of frequency band selection, electrocardiogram signal 13, transthoracic impedance variation signal 15 and pressure signal 16. Via a level regulating circuit 21, signal 13 is

sent to block 22 which, controlled by microprocessor 23 via known identification algorithms, compares the QRS-T sections on in-coming electrocardiogram signals with those considered to be normal, rejects those within the normal range, and sends to a processing and memorising block 24, controlled by microprocessor 23, any abnormal electrocardiogram signals indicating atrial or ventricular arrythmia, dynamic or temporary ischemic alterations (S-T section up or down), and second or third degree atrio-ventricular failure.

Electrocardiogram signal 13 and transthoracic impedance variation signal 15 are sent to block 25, which analyses the said impedance variation synchronous with electrocardiogram section QRS, to give a systolic output variation signal 26. As the reference systolic output value may be set individually, given real cardiac output (CO) measured simultaneously and directly (the best non-invasive method, in terms of accuracy and automation, is a $CO_2$ measurement), signal 26, via circuit block 27, supplies a signal 28 proportional to cardiac output and which is also sent to processing and memorising block 24. Signal 16 is also sent to block 25 which, by working out the value of the said signal synchronous with section QRS of electrocardiogram signal 13, gives a signal 29, as a function of tissue perfusion pressure, which is also sent to processing block 24.

Variations in transthoracic impedance signal 15 associated with breathing activity depend on breathing rate and current volume. Breathing rate times current volume gives ventilation/minute. The said signal 15 is therefore also sent to a processing block 30, which also receives a signal from a clock 31, and which detects the distance between peak breathing points to give a first signal 32, depending on breathing rate and expressing the number of breaths per minute, and a second signal 33 proportional to ventilation/minute and obtained by means of an integral function over one minute of the breathing impedance curve. Both signals 32 and 33 are sent to processing block 24, which also receives the signal from clock 31. The said processing block 24, controlled by microprocessor 23, analyses the in-coming signals relative to the said physiological variables, distinguishes between physiological and pathological variations, identifies various critical conditions, as described in more detail later on, activates an alarm block 34 which, as shown in Fig.1b, may comprise both optical and acoustic alarm means, 35 and 36, and memorises the values of the said critical physiological variables so detected. Furthermore, it may automatically clear the memory of any non-pathological physiological variables. Block 24 may conveniently provide for minimum seven days' data storage, the said second part 18 being supplied by a renewable or rechargeable long-life supply system. The said second part 18 also comprises a circuit block 37 comprising setting circuits and circuits for automatically scanning the signal reference values of the various physiological variables being controlled. The said block 37 controls blocks 21, 25, 30 and 27 and may conveniently be controlled by microprocessor 23. Processing block 24 therefore receives signals 13, 28, 32 and 33, which may be set for supplying real physiological variable values. Signal 29, however, proportional to tissue perfusion pressure, cannot be used for setting purposes,
when compared with pressure measured by a sphygmomanometer on the humeral artery. Variations in the said sig
nal 29, together with other in-coming data relative to
other physiological variables, are used, however, for
monitoring physiological behaviour (rise in perfusion
pressure, caused by physical strain, alongside increased
heart rate and cardiac output or vice versa) and for detecting pathological variations, such as : fall in perfusion pressure despite a simultaneous increase in heart
rate (tachyarrythmia), fall in perfusion pressure with
severe bradycardia, rise in perfusion pressure with no
accompanying increase in physical activity.

The control algorithm performed by processing block 24,
for detecting critical conditions, is based on a number
of syndromic associations useful in making the following diagnoses.

Physiological sinus tachycardia develops gradually, during muscular activity, and is proportional to work done,
oxygen consumption, ventilation/minute, cardiac output
and arterial pressure. Ventilation/minute increases along
side an increase in both breathing rate and current volume.

Sinus tachycardia resulting from neuropsychic stimulation is independent of motorial activity or oxygen consump
tion, is unaccompanied by increased ventilation/minute,
but may be accompanied by increased breathing rate alone,
or also increased arterial pressure. Cardiac output re-
mains unchanged.

Supraventricular tachycardia develops and ceases rapidly, with no change in ventilation/minute or breathing
rate. No change or a fall is registered in cardiac output (with a heart rate of over 150) and in arterial
pressure.

Ventricular tachycardia is accompanied by a fall in
cardiac output and arterial pressure; ventilation/minute
remains unchanged, whereas breathing rate may increase
in relation to cardiac insufficiency.

Ventricular fibrillation is accompanied by a sharp, massive fall in cardiac output and arterial pressure, followed, a few seconds later, by ventilation arrest. Unlike
ventricular fibrillation, asystolia resulting from heart
failure silences the signal.

In view of the foregoing considerations, the alarm conditions detected by processing block 24 are therefore
determined by : severe arrythmia, myocardiac ischemia,
acute left ventricular failure, each of which may either
develop gradually or appear suddenly. Each critical condition is classified by block 24 according to one of
three danger levels : level one : pre-alarm; level two:
alarm; level three : critical alarm; the signal issued
by optical and acoustic alarms 35 and 36 being differentiated accordingly.

Pre-alarm arrythmias are : highly frequent, polymorphous,
premature ventricular pulsations in pairs or clusters,
and second-degree atrio-ventricular failure.

Alarm arrythmias are : ventricular tachycardias, unsus-
tained or sustained up to 30 seconds, within a frequency
range of 130-180 beats/minute, and asystolias of three seconds or over, caused by sino-atrial or atrio-ventricular failure.

Critical alarm arrythmias are : sustained ventricular tachycardias of over 180 beats/minute, ventricular fibrillations and asystolias lasting over five seconds. Myocardiac ischemia triggers the pre-alarm when section S-T of electrocardiogram signal 13 rises or falls by 2 mm or more, for at least 15 seconds, on either branch of electrodes 6.

An ischemia alarm is triggered when the rise or fall in the said section S-T persists for over 15 seconds. The critical alarm is triggered when the said rise or fall in section S-T exceeds 4 mm, or when the said rise of fall is accompanied by first and second degree ventricular arrythmia and/or left ventricular failure. Pre-alarm conditions may also include changes in heart rate and arterial pressure accompanied by or preceding a rise or fall of even less than 2 mm in section S-T.

Left ventricular failure may be detected at the presymptomatic stage (level one alarm) by :

1) A sustained increase of over two minutes, as compared with reference values, in sinus heart rate (over 20' beats/minute), arterial pressure (over 30%) and breathing rate (over 5 breaths/minute) during resting hours (recorded by clock 31).

2) A sustained increase of over two minutes in breathing rate (over 5 breaths/minute) accompanied by a fall in cardiac output (over 10%).

3) An increase in heart rate accompanied by a fall in cardiac output during physical activity.

4) An increase in breathing rate unaccompanied by increased cardiac output in patients suffering from bradyarrythmia.

Left ventricular failure alarms of the second or third level are triggered by : persistent sinus tachycardia (over 110 beats/minute), tachypnea with no increase in volume/minute (over 20 breaths/minute), rise or fall in arterial pressure, and a fall in cardiac output as compared with reference values.

The said second part 18 also presents a socket 38 into which may be plugged a connector 39 on a third part 40, the latter being a unit installable, for example, in a hospital control centre and being, for example, a dynamic electrocardiogram reader designed to :

- receive the data stored in the memory on processing block 24;
- collect the said data on real-time tables or graphs;
- print out any interesting data on paper 41;
- monitor the signal from receiver 20 on fluorescent screen 42 for checking any misread physiological variables (false positives or negatives) or wrongly-formulated alarms;
- tune into the radio-frequency signal transmitted by transmitter 5 on first part 1, for checking and real-time setting each signal via microprocessor 23 on setting circuit 37.

The advantages of the unit according to the present invention will be clear from the foregoing description. In particular, the said first part 1 remains permanently inserted under the skin for continuously transmitting sig- nals to a portable pocket part, with no restriction in the activity of the patient whose conditions is thus controlled continuously and who is informed immediately, via alarm means on the portable pocket part, of any cri tical conditions, as early as the pre-alarm stage, thus enabling appropriate remedial or preventive action to be taken. Periodically transferring the data stored in the portable pocket part to the unit at the control centre enables an in-depth trend analysis to be conducted of the physiological cardiorespiratory variables being controlled. The unit according to the present invention also provides for greater efficiency in detecting criti cal conditions, on account of it detecting, not only an electrocardiogram signal, but also other physiological variables, such as ventilation/minute, systolic output, cardiac output and arterial pressure.

In addition to patients requiring constant care and observation, the unit according to the present invention may also be applied to subjects performing physical activities under particularly gruelling conditions, e.g. astronauts.

To those skilled in the art it will be clear that changes may be made to the embodiment as described herein without, however, departing from the scope of the present invention. For example, a few of the circuits for picking up basic signals 13, 15 and 16, from which to obtain signals 28, 29, 32 and 33 representing the physiological variables being controlled, may be formed on second part 18 instead of first part 1.

CLAIMS

1) - Unit for continuously monitoring physiological cardiorespiratory variables, characterised by the fact that it comprises a first subcutaneous part (1) having means (6, 9, 12) for detecting first physiological parameters, and means (7, 14, 17) for handling the said first parameters and transmitting relative first signals (13, 15, 16) to a second part (18) outside the patient's body; the latter part (18) having means (25, 30, 27, 22) for handling the said first signals (13, 15, 16), and means (24) for processing the said second physiological variables and/or variations in the same, received from the said handling means (25, 30, 27, 22), for detecting critical conditions and activating alarm means (34, 35, 36).

2) - Unit according to Claim 1, characterised by the fact that the said first part (1) comprises means (5) for radio-frequency transmitting the said first signals (13, 15, 16) to receiving means (20) on the said second part (18).

3) - Unit according to Claim 1 or 2, characterised by the fact that the said means for detecting the said first physiological parameters comprise at least two electrodes (6) for detecting a difference in potential and so providing a characteristic electrocardiogram signal (13), and for detecting transthoracic impedance for providing at least a few of the said second physiological variables.

4) - Unit according to Claim 3, characterised by the fact that it comprises three electrodes (6).

5) - Unit according to Claim 3 or 4, characterised by the fact that the said means for detecting the said first physiological parameters comprise means (9, 12) for detecting a pressure outside the said first part (1).

6) - Unit according to Claim 5, characterised by the fact that the said pressure detecting means comprise an electric pressure microtransducer (12) housed in the said first part (1) and connected hydraulically to a network of fluid-filled tubes (9) arranged about the said first part (1).

7) - Unit according to one of the foregoing Claims, characterised by the fact that the said first part (1) comprises an extremely long-life supply battery (3) and is housed in a titanium-plated case (2).

8) - Unit according to one of the foregoing Claims, characterised by the fact that the said second part (18) is pocket-portable.

9) - Unit according to one of the foregoing Claims, characterised by the fact that the said second physiological variables comprise at least some of the following : a characteristic electrocardiogram signal (QRS-T), ventilation/minute (VE), breathing rate, systolic output (SO), cardiac output (CO) and arterial pressure (AP).

10) - Unit according to one of the foregoing Claims, characterised by the fact that the said means (7, 17, 14, 25, 30, 27, 22) for handling the said first physiological parameters for obtaining the said second physiological variables are housed at least partially or totally in the said first (1) and/or the said second (18) part.

11) - Unit according to one of the foregoing Claims, characterised by the fact that the said processing means
(24) on the said second part (18) comprise a micropro-
cessor (23) and timing means (31).

12) - Unit according to one of the foregoing Claims, cha
racterised by the fact that the said second part (18)
comprises memory means controlled by the said processing
means (24).

13) - Unit according to one of the foregoing Claims, cha
racterised by the fact that the said processing means (24)
are designed to establish critical conditions of different
levels, and to differentiate activation of the said alarm
means (34, 35, 36) accordingly.

14) - Unit according to one of the foregoing Claims, characterised by the fact that it comprises a third part (40)
to which the said second part (18) may be connected for
transferring a large number of the said second physiological variables stored in the said second part (18).

15) - Unit according to Claim 14, characterised by the
fact that the said third part (40) comprises means for .
processing and/or analysing and/or printing (41) and/or
monitoring on a screen (42) the said second physiological
variables received from the said second part (18).

16) - Unit according to Claim 14 or 15, characterised by
the fact that the said third part (40) comprises means
for receiving the said signals from the said first part
(1) and for automatically setting the said handling means
(21, 25, 30, 27).

Fig.1a

Fig.1b

Fig.1c

0209804

Fig.2a

Fig.2b